**Europäisches Patentamt**

(19) **European Patent Office**    (11) Numéro de publication: **0 175 630**

**Office européen des brevets**                **B1**

(12)      # FASCICULE DE BREVET EUROPÉEN

(45) Date de publication du fascicule du brevet:     (51) Int. Cl.⁴: **A 61 M 15/00,** A 61 M 11/04
**31.05.89**

(21) Numéro de dépôt: **85440054.6**

(22) Date de dépôt: **19.09.85**

(54) **Appareil inhalateur destiné à la guérison des rhumes.**

(30) Priorité: **20.09.84 FR 8414410**

(43) Date de publication de la demande:
**26.03.86 Bulletin 86/13**

(45) Mention de la délivrance du brevet:
**31.05.89 Bulletin 89/22**

(84) Etats contractants désignés:
**AT BE CH DE GB IT LI LU NL SE**

(56) Documents cité:
**FR-A-960 469**
**FR-A-1 492 214**
**GB-A-526 678**

(73) Titulaire: **Le Pabic, Jean Pierre, 20 avenue des Acacias, F-92500 Rueil Malmaison (FR)**

(72) Inventeur: **Le Pabic, Jean Pierre, 20 avenue des Acacias, F-92500 Rueil Malmaison (FR)**

(74) Mandataire: **Nuss, Pierre, 10, rue Jacques Kablé, F-67000 Strasbourg (FR)**

EP 0 175 630 B1

LIBER, STOCKHOLM 1989

## Description

La présente invention concerne un appareil inhalateur destiné à la guérison des rhumes en application des récentes découvertes du professeur A. LWOFF, découvertes qui ont été largement diffusées.

Il existe déjà des appareils destinés à guérir les rhumes en application de ces découvertes, mais ils nécessitent des moyens importants, en particulier de l'air comprimé, des systèmes de régulation de la température et du degré hydrométrique et sont donc d'un prix élevé.

Au contraire, la présente invention est de conception extrêmement simple et donc du prix d'un objet de grande diffusion.

Selon les découvertes du professeur A. LWOFF, le malade doit respirer de l'air chaud et saturé d'humidité pendant trois séances de 30 mn, les séances étant espacées de deux heures. La température de cet air chaud et humide se situe idéalement à 43° C. En effet, cette température est largement suffisante pour que le traitement soit efficace et elle est supportée sans inconvénient par les utilisateurs.

Pour répondre à ces exigences, un inhalateur simple dans lequel on verse de l'eau préalablement chauffée n'est pas adéquat étant donné que la durée de l'inhalation est trop faible, d'une part, et que, d'autre part, la température de l'air humide respiré est trop forte au début ce qui peut occasionner des brûlures, et trop faible par la suite, ce qui est inefficace.

Il existe également des inhalateurs contenant un système de chauffage, mais ces inhalateurs ne sont pas prévus pour une telle utilisation étant donné que la puissance fournie n'est pas adaptée à cet usage et qu'ils délivrent ainsi un air humide dont la température se situe hors des tolérances nécessaires pour le traitement. D'autre part, ils ne contiennent pas de moyen de saturer l'air en humidité.

On connaît par GB-A-526 678 un inhalateur dans lequel de l'air chaud est produit, cet air chaud permettant l'évaporation d'une substance en la chauffant.

On connaît également par FR-A-960 469 un inhalateur dans lequel l'air s'échauffe à la surface de l'eau chaude.

Mais ces deux dispositifs ne permettent ni de saturer l'air en humidité, ni de maintenir sa température constante et indépendante du débit respiratoire du malade. En effet, FR-A-960 469 n'autorise aucun réglage, ni de la température de l'air à la sortie de l'inhalateur, ni du débit de vapeur d'eau, ces deux paramètres restant incontrôlables. Quant à GB-A-526 678, il ne permet qu'un réglage grossier (trois positions) de la température de l'air à la sortie de l'inhalateur et ne permets pas non plus un réglage d'un débit de vapeur d'eau. Dans ces deux dispositifs, il n'y a donc aucune relation directe entre la variation du débit de vapeur d'eau et la température de l'air inspiré par le malade.

Au contraire, la présente invention est conçue de telle façon que l'air respiré soit, d'une part, saturé en humidité et, d'autre part, que l'énergie qui lui est apportée puisse être réglée par le malade de façon à obtenir la température voulue quel que soit son débit respiratoire.

L'appareil inhalateur conforme à l'invention présente, en effet, une chambre (7) qui communique par son extrémité supérieure avec une chambre (9) dont le volume est supérieur au volume d'air inspiré par le patient en une seule fois, de manière à permettre un mélange entre la vapeur d'eau, produite par ébullition et constituant l' apport réglable d'énergie, avec l'air provenant de l'extérieur par les orifices d'entrée d'air (12) situées à la base de la dite chambre de mélange (9) sous l'effet de l'inspiration du malade, les deux chambres (7) et (9) étant séparées par un élément (6), muni d'orifice (4) de passage de la vapeur d'eau empêchant tôut contact direct entre l'air contenu dans l'appareil et l'eau en ébullition, un moyen de réglage (16, 3) permettant, en outre, d'assurer la saturation en humidité et le chauffage à la température désirée de ce mélange air/vapeur d'eau grâce à une régulation faite par l'utilisateur lui-même en fonction de sa sensation de la température.

La chambre (9) est limitée en haut par une paroi conique (8) présentant à sa base des orifices d'entrée d'air (12) permettant l'introduction du flux d'air ambiant dans la chambre (9) sous l'effet de l'inspiration du patient, et à sa partie supérieure un orifice (13) où le patient introduit le nez pour inspirer l'air humidifié et chauffé.

Les calculs suivants vont permettre de montrer que l'air ainsi respiré est saturé en humidité. On calculera, d'autre part, la puissance mise en jeu par le dispositif ainsi que la quantité d'eau consommée au cours d'une séance de 30 mn.

Il est supposé que la température ambiante est de 20° C ce qui est généralement le cas des appartements chauffés en hiver et la quantité d'eau contenue dans l'air est dans un premier temps négligée.

A 43° C, la pression de vapeur saturante de l'eau est égale à 64,8 mm de mercure soit 8,5 % en volume.

Le poids de la mole d'eau étant 18 g, tandis que celle de l'air étant 29 g, cela donne les proportions suivantes en poids:

5,45 % d'eau, 94,55 % d'air

La chaleur spécifique de l'air étant égale à $1 J/g \times ° C$, la quantité de chaleur nécessaire pour élever 94,55 g d'air de 20° C à 43° C est égale à:

$$94,55 \times 1 \times (43 - 23) = 2175 \text{ j}$$

La chaleur spécifique de la vapeur d'eau étant égale à $1,9 J/g \times ° C$, la quantité de chaleur fournie par l'abaissement de 5,45 g d'eau de 100 C à 43° C est égale à:

$$5,45 \times 1,9 \times (100 - 43) = 590 \text{ J}$$

Cette énergie est plus faible que celle qui est nécessaire pour élever 94,55 g d'air de 20°C à 43°C. Le complément doit donc provenir nécessairement de la condensation de l'eau, ce qui assure la saturation.

Etant donné que l'énergie manquante est égale à:

$$2175 - 590 = 1585 \text{ j}$$

et que la chaleur latente de vaporisation de l'eau à 43°C est égale à 2410 J/g, il faudra vaporiser $1585/(1,9 \times 57 + 2410) = 0,63$ g d'eau supplémentaire pour obtenir 100 g d'air à 43°C saturé en humidite.

La quantité d'eau totale ainsi calculée est donc égale à:

$$5,45 + 0,63 = 6,08 \text{ g}.$$

Ces résultats sont à présent rapportés au volume d'air respiré par un utilisateur, ce qui se situe sensiblement autour de 12 l/mn.

La mole d'air ayant un volume de 22,4 l à 0°C et un poids de 29 g, 94,55 g d'air représentent un volume de:

$$94,55 \times (22,4/29) \times (1 + 43/273) = 84,5 \text{ l}$$

Le même calcul rapporté à l'eau qui a un poids molaire de 18 g donne:

$$5,45 \times (22,4/18) \times (1 + 43/273) = 7,9 \text{ l}$$

On obtient ainsi le volume total d'air humide:

$$84,5 + 7,9 = 92,4 \text{ l}$$

L'énergie dépensée a été consommée par la vaporisation à 100°C de 6,08 g d'eau.

La chaleur latente de vaporisation de l'eau à 100°C étant égale à 2245 J/g, cette énergie est égale à:

$$6,08 \times 2245 = 13650 \text{ J}$$

Le temps nécessaire à la consommation de 92,4 l d'air est égal à:

$$92 \times 60/12 = 460 \text{ s}$$

La puissance nécessaire est donc:

$$13650/460 = 30 \text{ W}$$

correspondant à une quantité de vapeur produite par seconde égale à:

$$6,1/460 = 13 \times 10^{-3} \text{ g/s}$$

La quantité d'eau totale consommée pendant une séance de 30 mn est donc égale à:

$$13 \times 10^{-3} \times 30 \times 60 = 23 \text{ g}$$

Naturellement, la quantité de vapeur produite doit être ajustée en fonction du débit respiratoire de l'utilisateur. Dans une première forme de réalisation, le générateur de vapeur possède une ouverture supplémentaire vers l'extérieur (15), de façon à ce que, cette ouverture étant réglable par l'utilisateur, elle évacue l'excédent de vapeur produite. Il y a alors lieu de prévoir une puissance sensiblement supérieure à ce qui a été calculé précédemment de façon à couvrir le cas de n'importe quel utilisateur, par exemple 40 W.

L'usager procède alors au réglage de façon à ce que l'air humide respiré soit le plus chaud possible sans provoquer de sensation de brûlure.

Dans une seconde forme de réalisation, où le générateur de vapeur est alimenté en énergie électrique, le réglage de la quantité de vapeur produite se fait par un variateur de puissance (3) interposé sur le câble d'arrivée du courant. L'usager procède au réglage selon le même critère que précédemment.

Dans ce cas, il est possible de prévoir une puissance maximale très supérieure au besoin de production de vapeur, par exemple 300 W, de façon à raccourcir considérablement la durée d'échauffement de l'eau contenue dans le générateur de vapeur. En effet, pour échauffer 100 g d'eau de 20°C à 100°C à l'aide d'une puissance de 300 W il faut:

$$100 \times (100 - 20) \times 4,18/300 = 111 \text{ s}$$

soit 1 mn 51 s, tandis qu'avec une puissance de 40 W, il faut:

$$100 \times (100 - 20) \times 4,18/40 = 836 \text{ s}$$

soit 13 mn 56 s.

Ces possibilités de réglage de l'une ou l'autre forme de réalisation couvrent, en outre, d'autres facteurs pouvant influer sur la température de l'air inspiré. Ce sont:

- La température de l'air ambiant:

Les calculs précédents montrent que lorsque l'air est plus chaud, la quantité de vapeur excédentaire dont la condensation provoque l'échauffement final doit être réduite.

- L'humidité relative de l'air ambiant:

Plus cette humidité est élevée, plus la quantité de vapeur à produire sera faible.

- Les pertes calorifiques par les parois de l'inhalateur:

Dans une forme de réalisation où le générateur de vapeur n'est pas calorifugé, ces pertes ne sont pas du tout négligeables et il y a lieu de prévoir une puissance supplémentaire d'environ 20 W, ce qui porte à 60 W la puissance minimum à prévoir.

Selon une autre caractéristique de l'invention, le volume de la chambre de mélange (9) où a lieu le mélange air-vapeur avant inspiration doit être assez grand et en particulier supérieur au volume d'air correspondant à une inspiration.

Si tel n'était pas le cas, le fait que le passage de l'air à travers cette chambre (9) n'est pas régulier, car il suit le rythme respiratoire de l'utilisateur, provoquerait un mélange irrégulier de l'air et de la vapeur entraînant des variations de température inopportunes. Au contraire, si le volume de ladite chambre (9) est suffisant, par exemple 1 l, à chaque inspiration une partie de l'air chauffé et humidifié est remplacée par de l'air froid et sec qui s'échauffe et se sature pendant l'expiration.

Selon une forme de réalisation préférentielle, le générateur de vapeur est constitué d'une chambre dite chambre d évaporation (7) de volume réduit, par exemple 0,1 l destiné a recevoir l'eau à évaporer et contenant une résistance immergeable (5) connectée à une source d'électricité.

Cette chambre (7) est fermée à l'extrémité supérieure par un élément (6) possédant des orifices (11) à travers lesquels la vapeur passe dans la chambre de mélange (9).

Etant donné le faible volume de la chambre d'évaporation (7) et le fait que celle-ci doit se trouver à la partie inférieure de l'inhalateur, il pourrait s'ensuivre des problèmes de stabilité quand celui-ci est posé sur une surface horizontale.

C'est pourquoi cette chambre (7) est rendue solidaire d'un cylindre de plus grand diamètre assurant une bonne stabilité et sur lequel vient s'emboiter à la fois l'élément (6) de la chambre d'évaporation (7) et paroi (8) de la chambre de mélange (9).

Cette enveloppe (8) est constituée par un cône de révolution positionné base en bas et à l'extrémité supérieure duquel se trouve l'orifice (13) dans lequel le malade introduit le nez. De façon à accroître à la fois l'étanchéité et le confort d'utilisation, les bords de l'ouverture sont munis d'un bourrelet (14).

Sur la partie inférieure sont prévus les trous (12) d'entrée de l'air ambiant.

La description suivante, en regard des dessins annexés, le tout donné à titre d' exemple non limitatif, fera bien comprendre comment l'invention peut être réalisée.

Les dessins représentent:

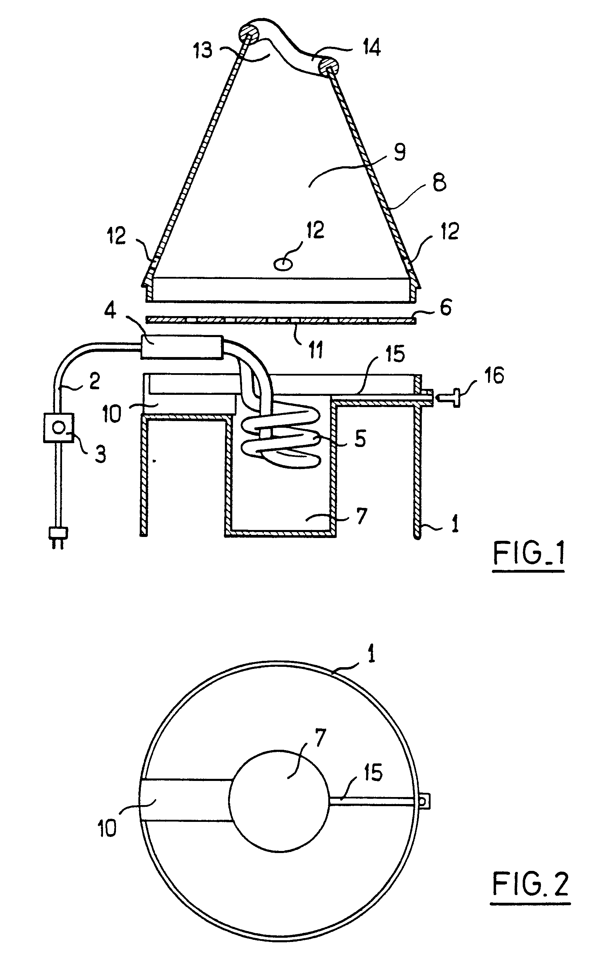

A la figure 1: les différentes parties de l'inhalateur à savoir:

-Une coupe selon un plan vertical du socle (1) de l'inhalateur.

- L'ensemble cordon d'alimentation (2), variateur de puissance (3) équipé dans une forme de réalisation, manchon (4) et résistance (5).
- Une coupe selon un plan vertical de l'élément (6) de la chambre d'évaporation (7).
- Une coupe selon un plan vertical de la paroi (8) de la chambre de mélange (9).

A la figure 2 une vue de dessus du socle (1).

Le manchon (4) de la résistance (5) vient se placer dans le conduit (10) du socle (1) de l'inhalateur, ce qui a pour effet de positionner la résistance (5) dans la chambre d'évaporation (7). L'utilisateur y verse l'eau destiné à produire la vapeur.

Ses dimensions, égales à 2,5 cm pour le rayon intérieur et 6 cm pour la hauteur font que sa contenance est d'environ 0,1 l. L'élément (6) de la chambre d'évaporation (7) se place à l'intérieur des parois du socle (1) au-dessus de cette chambre (7). Il y est maintenu par la paroi (8) de la chambre de mélange (9) qui s'encastre à l'intérieur du socle (1) et bloque l'ensemble.

Des trous (11) dans l'élément (6) de la chambre d'évaporation (7) permettent à la vapeur d'être injectée dans la chambre de mélange (9).

Des trous (12) situés en bas de la paroi (8) de la chambre de mélange (9) permettent à l'air extérieur d'y pénétrer lors de l'inspiration de l'utilisateur. Celui-ci se place le nez dans l'orifice supérieur (13) doté d'un bourrelet (14).

Selon une forme de réalisation, un conduit (15) met en communication la chambre d'évaporation (7) et l'extérieur. Ce conduit peut être plus ou moins obturé en agissant sur la vis (16).

L'appareil inhalateur selon l'invention est particulièrement destiné à la guérison des rhumes.

## Revendications

1. Appareil inhalateur destiné à la production d'air chaud et humide pour le traitement des rhumes, comprenant un générateur de vapeur, sous la forme d'une chambre d'évaporation (7) contenant une source de chaleur (5) avec un apport réglable d'énergie, de l'air étant aspiré de l'extérieur par des orifices d'entrée d'air, appareil caractérisé en ce que la chambre d'évaporation (7) communique par son extrémité supérieure avec une chambre de mélange air/vapeur (9) dont le volume est supérieur au volume d'air inspiré par le patient en une seule fois, de manière à permettre un mélange entre la vapeur d'eau, produite par ébullition et constituant l'apport réglable d'énergie, avec l'air provenant de l'extérieur par les orifices d'entrée d'air (12) situées à la base de la dite chambre de mélange sous l'effet de l'inspiration du malade, les deux chambres (7) et (9) étant séparées par un élement (6), muni d'orifices (11) de passage de la vapeur d'eau, empêchant tout contact direct entre l'air contenu dans l'appareil et l'eau en ébullition, un moyen de réglage (16, 3) permettant, en outre, d'assurer la saturation en humidité et le chauffage à la température désirée de ce mélange air/vapeur d'eau grâce à une régulation faite par l'utilisateur lui-même en fonction de sa sensation de la température.

2. Appareil inhalateur, selon la revendication 1, caractérisé en ce que la chambre (9) est limitée en haut par une paroi conique (8) présentant à sa base les dites orifices d'entrée d'air (12) permettant l'introduction du flux d'air ambiant dans la chambre (9) sous l'effet de l'inspiration du patient, et, à sa partie supérieure, un orifice (13) où le patient introduit le nez pour inspirer l'air humidifié et chauffé.

3. Appareil inhalateur, selon l'une quelconque des revendications 1 et 2, caractérisé en ce que le moyen de réglage du débit de vapeur d'eau comprend un conduit (15) d'évacuation d'une partie de la vapeur produite dans l'atmosphère, mettant en communication la chambre d'évaporation (7) et l'extérieur, ce conduit (15) pouvant être plus ou moins obturé par tout moyen connu, par exemple, en agissant sur une vis (16).

4. Appareil inhalateur, selon l'une quelconque des revendications 1 et 2, caractérisé en ce que le générateur de vapeur étant alimenté en énergie électrique, le moyen de réglage peut agir directement sur la puissance fournie en étant, par exemple, intercalé dans le câble d'alimentation (2).

5. Appareil inhalateur, selon la revendication 4, caractérisé en ce que le moyen de réglage de la puissance est un variateur électronique de puissance (3).

6. Appareil inhalateur, selon l'une quelconque des revendications 4 et 5, dont la puissance maximale est très supérieure au besoin en fonctionnement normal.

7. Appareil inhalateur, selon l'une quelconque des revendications 1 à 6, caractérisé en ce que la chambre (7) avantageusement cylindrique est rendue solidaire d'un socle (1) de plus grand diamètre assurant une bonne stabilité, et sur lequel vient s'emboîter à la fois l'élément (6) de la chambre d'évaporation (7) et la paroi (8) de la chambre de mélange (9).

8. Appareil inhalateur, selon l'une quelconque des revendications 1 à 7, caractérisé en ce que l'orifice d'inspiration (13) de l'air humidifié et chauffé est muni d'un bourrelet (14).

9. Appareil inhalateur, selon la revendication 1, caractérisé en ce que la source de chaleur (5) est sous la forme d'une résistance immergeable.

10. Appareil inhalateur, selon la revendication 1, caractérisé en ce que le générateur de vapeur est intégré dans un socle (1) de diamètre plus grand que celui de la dite chambre d'evaporation (7).

**Patentansprüche**

1. Inhalationsapparat zur Erzeugung warmer und feuchter Luft zur Heilung von Schnupfen, bestehend aus einem Dampferzeuger in Form einer eine Heizquelle (5) enthaltenden Verdampfkammer (7), mit einer einstellbaren Energiezufuhr, wobei Luft von außen durch Lufteintrittsöffnungen angesaugt wird, dadurch gekennzeichnet, daß die Verdampfkammer (7) an ihrem oberen Ende mit einer Luftmischkammer (9) in Verbindung steht, deren Volumen größer als das von dem Patienten bei einem Mal eingeatmete Luftvolumen ist, um eine Mischung zwischen dem durch Sieden erzeugten und die regulierbare Energiezufuhr darstellenden Wasserdampf, mit der von außen, infolge des Einatmens des Kranken, durch die am Boden der besagten Mischkammer sitzenden Lufteintrittsöffnungen (12) kommenden Luft zu ermöglichen, wobei die beiden Kammern (7) und (9) durch ein mit Öffnungen (11) für den Durchfluß des Wasserdampfes versehenes Element (6) getrennt sind, welches jeden direkten Kontakt zwischen der in dem Apparat enthaltenen Luft und dem siedenden Wasser verhindert, wobei ein Reguliermittel (16, 3) außerdem die Sättigung mit Feuchtigkeit und die Aufheizung auf die gewünschte Temperatur dieser Luft/Wasserdampfmischung dank einer durch den Benutzer selbst je nach seinem Temperaturempfinden durchgeführten Einstellung sicherstellt.

2. Inhalationsapparat nach Anspruch 1, dadurch gekennzeichnet, daß die Kammer (9) oben durch eine konische Wand (8) begrenzt wird, die an ihrem Boden die besagten Lufteintrittsöffnungen (12), welche den Eintritt der Umgebungsluft infolge des Einatmens des Patienten ermöglichen, und an ihrem Oberteil eine Öffnung (13), in welche der Patient die Nase hineinsteckt, um die angefeuchtete und erhitzte Luft einzuatmen, aufweist.

3. Inhalationsapparat nach einem der Ansprüche 1 und 2, dadurch gekennzeichnet, daß das Reguliermittel für die Wasserdampfleistung eine Leitung (15) zum Ablassen eines Teils des erzeugten Dampfes in die Atmosphäre enthält, welche die Verdampfkammer (7) und die Außenluft verbindet, wobei diese Leitung (15) mehr oder weniger durch jedes bekannte Mittel, zum Beispiel durch betätigen einer Schraube (16) verschlossen werden kann.

4. Inhalationsapparat nach einem der Ansprüche 1 und 2, dadurch gekennzeichnet, daß der Dampferzeuger mit Strom gespeist wird, wobei das Reguliermittel direkt auf die gelieferte Leistung einwirken kann, indem es, zum Beispiel, bei dem Speisekabel (2) zwischengeschaltet wird.

5. Inhalationsapparat nach Anspruch 4, dadurch gekennzeichnet, daß das Reguliermittel für die Leistung aus einem elektronischen Leistungswandler (3) besteht.

6. Inhalationsapparat nach einem der Ansprüche 4 und 5, bei welchem die Höchstleistung viel höher über dem Bedarf bei Normalbetrieb liegt.

7. Inhalationsapparat nach einem der Ansprüche 1 bis 6, dadurch gekennzeichnet, daß die Kammer (7) vorteilhaft zylindrisch und mit einem Sockel (1) mit viel größerem Durchmesser fest verbunden ist, welcher eine gute Stabilität gewährleistet, und auf welchem gleichzeitig das Element (6) der Verdampfkammer (7) und die Wandung (8) der Mischkammer (9) aufgesteckt werden.

8. Inhalationsapparat nach einem der Ansprüche 1 bis 7, dadurch gekennzeichnet, daß die Öffnung (13) zum Einatmen von angefeuchteter und erwärmter Luft mit einem Wulst (14) versehen ist.

9. Inhalationsapparat nach Anspruch 1, dadurch gekennzeichnet, daß die Wärmequelle (5) als eintauchbarer Widerstand ausgebildet ist.

10. Inhalationsapparat nach Anspruch 1, dadurch gekennzeichnet, daß der Dampferzeuger in dem Sockel (1) mit größerem Durchmesser als dem der besagten Verdampfkammer (7) integriert ist.

## Claims

1. Inhaler for producing hot, humid air for the treatment of colds, comprising a vapour generator in the form of an evaporation chamber (7) containing a heat source (5) with a controllable energy supply, air being aspirated from the exterior through air inlet orifices, said inhaler being characterised in that the evaporation chamber (7) communicates via its upper end with a chamber (9) for mixing air and vapour, the volume of which is greater than the volume of air breathed in by the patient at one time, so as to allow the steam produced by boiling and constituting the controllable energy supply to be mixed with the air originating from the exterior via the air inlet orifices (12) located at the bottom of said mixing chamber as the patient breathes in, the two chambers (7) and (9) being separated by a member (6) which is provided with orifices (11) for the passage of steam and prevents direct contact between the air contained in the apparatus and the boiling water, a control means (16, 3) also allowing this mixture of air and steam to be saturated with humidity and heated to the desired temperature as the result of an adjustment made by the user himself as a function of the temperature felt by him.

2. Inhaler according to Claim 1, characterised in that the chamber (9) is limited at the top by a conical wall (8) having, at its bottom, said air inlet orifices (12) for introducing the flow of ambient air into the chamber (9) as the patient breathes in and, at its top, an orifice (13) where the patient inserts his nose in order to breathe in the humidified, heated air.

3. Inhaler according to any one of Claims 1 and 2, characterised in that the means for controlling the flow of steam comprises a tube (15) for discharging a proportion of the vapour produced into the atmosphere by enabling the evaporation chamber (7) to communicate with the exterior, this tube (15) being blockable to a greater or lesser extent by any known means, for example by acting on a screw (16).

4. Inhaler according to any one of Claims 1 and 2, characterised in that, since the steam generator is supplied with electric energy, the control means can act directly on the power supplied if it is, for example, inserted in the supply cable (2).

5. Inhaler according to Claim 4, characterised in that the means for controlling the power is an electronic power varying device (3).

6. Inhaler according to any one of Claims 4 and 5, the maximum power of which is much higher than necessary in normal operation.

7. Inhaler according to any one of Claims 1 to 6, characterised in that the chamber (7) which is advantageously cylindrical is connected to a base (1) of greater diameter which ensures good stability and on which the member (6) of the evaporation chamber (7) and the wall (8) of the mixing chamber (9) are both fitted.

8. Inhaler according to any one of Claims 1 to 7, characterised in that the orifice (13) for the breathing in of humidified, heated air is provided with a rim (14).

9. Inhaler according to Claim 1, characterised in that the heat source (5) is in the form of an immersible heating element.

10. Inhaler according to Claim 1, characterised in that the steam generator is integrated in a base (1) having a greater diameter than said evaporation chamber (7).

EP 0 175 630 B1

FIG.1

FIG.2

1